# EUROPEAN PATENT SPECIFICATION

(11) **EP 1 349 526 B1**
(45) Date of publication and mention of the grant of the patent: **01.03.2006**
(21) Application number: 01270294.0
(22) Date of filing: 12.12.2001
(51) Int. Cl.: A61F 13/56

(54) **FASTENING MEANS FOR AN ABSORBENT ARTICLE AND AN ABSORBENT ARTICLE**
BEFESTIGUNGSMITTEL FÜR EINEN SAUGFÄHIGEN ARTIKEL UND SAUGFÄHIGER ARTIKEL
SYSTEME DE FIXATION POUR ARTICLE ABSORBANT ET ARTICLE ABSORBANT

(30) Priority: 14.12.2000 SE 0004646
(43) Date of publication of application: 08.10.2003
(73) Proprietor: SCA Hygiene Products AB, 405 03 Göteborg (SE)
(72) Inventor: KLING, Roberg, S-511 63 Skene (SE); JÖNBRINK, Anna-Karin, S-443 51 Lerum (SE); JOHANSSON, Marie, S-435 37 Mölnlycke (SE); BLENNAR, Anna, FR-59910 BONDUES (FR)
(74) Representative: Egeröd, Lisbeth
(86) International application number: PCT/SE2001/002765
(87) International publication number: WO 2002/047597

(56) References cited:
- WO-A2-00/37009
- GB-A- 2 315 402
- US-A- 5 782 819
- US-A- 6 140 551

## Description

### Technical field

The present invention relates to a fastening means for fastening of an absorbent article, such as a diaper, incontinence guard or the like, on a user and an absorbent article comprising such fastening means.

### Background

Fastening means for absorbent articles often consist of so-called fastening flaps or fastening tabs, being arranged on the rear side portions of the article and of a reception portion arranged on the front portion of the article. A fastening means is arranged on the fastening tab, such as for example a hook or a loop portion of a hook and loop type of fastener and during fastening of the article around a wearer, the article is wrapped around the wearers body and fastening means of the fastening tabs is attached on the reception portion on the front side of the article. Examples of fastening tabs is disclosed in GB 2 311 096 B, WO 95/16425 A2, WO 96/19960 A1, US 5,738,930 A, EP 0 235 014 B1 and EP 0 321 234 A1.

One problem with today's fastening means is that the tab usually is made of a, in this respect, rather stiff material. As the wearer moves and consequently putting stress on the absorbent article, this kind of fastening tabs may cause skin irritations or sores on the user. Another problem is that the fastening means often not provide enough stability to the absorbent article around the wearer such that the article has a tendency to twist itself or loosening itself around the waist.

### Description of the invention

The object of the present invention is to solve the above-mentioned problem and to design an absorbent article exhibiting a stable fit around the wearer.

An absorbent article according to the present invention has an essentially elongated shape exhibiting a longitudinal direction and a transversal direction and exhibits two side edges extending along the longitudinal direction, one front end edge extending in the transversal direction, one rear end edge extending in the transversal direction, and a front portion, a rear portion and a crotch portion arranged therebetween. The article comprises a lower liquid impervious back sheet faced from the wearer, an upper liquid permeable top sheet faced against the wearer and an absorption core arranged between said sheets. The absorption core is elongated in the longitudinal direction and constitute together with the directly adjacent portions of the back sheet and the top sheet one in the longitudinal direction extending central portion. On each side of the central portion, the article exhibits front and rear side portions, respectively, at the front and rear portions, respectively, which preferably are elastic to offer a good comfort and a good fit about the wearer. If the absorbent article is a so-called belted diaper, the side portions may also consist of belt portions holding the article around the wearer. It is also possible to provide the article with both side portions and belt portions.

The liquid impervious back sheet of the absorbent article may for example, be made of a thin film of polyethylene (PE), polypropylene (PP) or other suitable materials, a hydrophobic nonwoven layer or a laminate of a thin film and a nonwoven material. This kind of laminate is often used to accomplish a soft and fabric like outside of the back sheet. To achieve a more airy and comfortable article, it is also possible to use a breathable back sheet preventing liquid to penetrate through the absorbent article but allows moisture to be exhausted from the article. This breathable back sheet can consist of single material layers or of a laminate of e.g., blown or moulded polyethylene films being laminated by e.g., a nonwoven layer of spunbond or spunbond-meltblown-spunbond (SMS).

The liquid permeable top sheet is preferably made of a material, which exhibits properties such as dryness and softness during use of the absorbent article, since this layer lies close to the user's body. It is desirable that the layer has a soft and fabric-like surface remaining dry even after repeating wettings. The top sheet can for instance consist of a nonwoven material having a soft and smooth surface such as e.g., a spunbond from polypropylene fibres. To keep the surface next to the skin of the user dry, a hydrophobic nonwoven material can be used, being perforated creating openings in the material being larger than the cavities between the fibres in the material. In such way, liquid can be directed through the perforated openings in the top sheet down to the underlying absorption core. Other examples of material suitable for use in the top sheet can for example be perforated plastic films such as for example a perforated polyethylene film. The top sheet can be bound to the underlying back sheet and with the absorption core, e.g., using adhesive or by any thermal bonding.

The absorption core is usually made of one or more layers of cellulose fibres, e.g., cellulose fluff pulp. Other materials that can be used are for example, absorbent nonwoven material, foam material, synthetic fibre material or peat. Except cellulose fibres or other absorbent materials, the absorption core may also comprise super absorbent material, so called SAP (super absorbent polymers), that is material in fibre form, particles, granules, film or the like, having the ability to absorb liquid corresponding to multiple times the own weight of the super absorbent material. The super absorbent material binds the liquid and forms a water containing gel. Further, the absorption core may comprise binder materials, shape stabilisation components or the like. Additional absorption layers improving the absorption properties may also be used, such as different types of liquid spreading material layers or inserts, so-called pads. The absorption core can be chemically or physically treated in order to improve the absorption properties. It is for instance possible to provide absorption layer using compressions in order to control the liquid flow in the absorbent core. It is also possible to enclose the absorbent layer(s) in a shell of for example tissue material.

The absorbent core usually has an elongated shape in the longitudinal direction and can for instance be essentially rectangular, T-shaped or hourglass shaped. In a T-shaped absorbent core, the transversal portion is intended to be faced against the front portion of the absorbent article during use, as it is placed in the area around the bowel of the user during use. A hour-glass shaped absorbent core is wider at the front and rear portions than in the crotch portion, at the same time as the design facilitates for the article to adapt itself and fit tightly about the wearer.

The portions being arranged on each side of the central portion extending outside the absorbent core and between the rear and front side portion is preferably provided with one or more elastic means, at least in the portion of the absorbent article forming the crotch portion during use. The elastic means functions as a leg elastic and has a task to prevent liquid and faeces leaking out through the longitudinal edges and in this way forms outer liquid barriers. The elastic means can consist of one or more elastic threads being applied in a prestretched condition between the back sheet and the top sheet or between the back sheet and a separate layer outside the top sheet in the transversal direction. The separate layer may consist of a ribbon extending along the absorbent core and the longitudinal edges. Alternatively, the elastic means may be arranged between the layers in a relaxed condition and instead both layers are then stretched upon application. It is also possible to arrange the elastic means on the outside of the back sheet or on the inside of the top sheet.

For further prevention of liquid or faeces leaking out, the absorbent article can also be provided with inner liquid barriers being attached in connection to the longitudinal edges inside the outer barriers. The inner barriers is preferably made of an essentially liquid impervious material, such as for instance a hydrophobic nonwoven or a plastic film and is designed as a longitudinal web, having a first edge being connected to the absorbent article and a second free edge intended to lye close to the user during usage of the absorbent article. The other edge is provided with one or more elastic elements, preferable an elastic thread, which in a contracted condition contract the free edge creating a standing barrier. The inner barrier can be designed as a ribbon of a single layer wherein the free edge is folded to enclose the elastic element in order to prevent direct contact of the elastic thread against the wearer. Alternatively, the barrier can be constructed using two joined layers with the elastic thread attached at the edge of the free end between the both layers. Here, the inner layer of the barrier can consist of an extension of the top sheet and the outer layer of an essentially liquid impervious material, or the inner and outer layer of the barrier may consist of one and same material ribbon being folded around the elastic thread.

The rear and/or the front portions of the article can also be provided with a so called waist elastic consisting of elastic means arranged along the end edges of the front and/or rear portions in order to provide a soft and flexible enclosure around the waist of the user. The elastic means are suitably attached between the back sheet and the top sheet using an adhesive or by welding, e.g., ultrasonic welding. The elastic means may consist of one or more elastic threads being applied in a prestretched condition and in this way forms a waist elastic. Alternatively, the elastic means may be applied between the layers in a relaxed condition and instead are then both layers stretched upon application. Another common variant of elastic element is elastic foam materials consisting of a thin ribbon of e.g., polymetene foam, which can be applied between both layers in the same way as the elastic threads. Naturally, it is also possible to arrange the elastic means for the waist elastic on the outside of the back sheet or on the inside of the top sheet.

At least one soft and inelastic fastening tab is arranged on the front or rear side portions of the absorbent article in order to attach the absorbent article around a wearer. The fastening tab is intended to join the rear and front portions of the absorbent article in that the fastening means, arranged at the fastening tab, can attach against a reception portion for the fastening means, arranged at the front or rear portion. Preferably, the article is provided with two fastening tabs arranged at the rear side portions, a fastening tab on each rear side portion, and one reception portion at the front portion of the article.

The fastening tabs are made of a very soft and inelastic material such as a single nonwoven layer, a laminate or any other suitable material, in order to not cause skin irritations or sores on the user. The fastening tabs also functions as a carrier for the fastening means. By a very soft material, it means that it exhibits a bending stiffness, the so-called "Gurley stiffness", which not exceeds 100 mg, preferably 75 mg.

One suitable method for the measurement of the Gurley stiffness is described in the TAPPI standard test T 543 om-94 (Bending resistance of paper (Gurley-type stiffness tester)). The measurements are performed in a so-called "Gurley-type tester" and the stiffness for a plurality of different materials can be tested using the method. The stiffness is measured in the unit milligrams (mg) or in the identical unit "Gurley units".

The method comprises the measurement of the force required to bend a test sample according to the test method.

The material being inelastic means that the possibility to stretch out the material is limited. A material named inelastic in this context, does not exhibit a level of elasticity that allows it to be extended more than 25 % of its relaxed, relaxed length, i.e., it is impossible to stretch it to a length being 1.25 times the relaxed length without destroying or breaking the material

The fastening mean may consist of a tape tab, a hook or loop portion of a hook and loop type fastener or any other form of mechanical fastening means, such as for example push buttons or the like. The fastening means is attached on the fastening tab, for example using adhesive, tape, thermal bonding or any other suitable fastening agent, on the part of the fastening tab which is faced away from the side portions of the article and on the side of the fastening tab which is faced against the reception portion during usage of the article.

The reception portion for the fastening tab is suitably designed as a ribbon of a reception material extending essentially parallel to the front-end edge. In that case, the reception material can be designed from a plastic ribbon being suitable for the reception of an adherent tape tab or a loop or hook portion of a hook and loop type fastener for a mechanical fastening means. The American patent document US 5,024,672 A describes an absorbent article having adherent tape tabs. Alternatively, the reception portion can consist of two or more parts for the reception of the fastening means, such as for example two or more ribbons arranged adjacent to each other either in the transversal direction or in the longitudinal direction. If push buttons are used as fastening means, then the reception portion can consist of one or more receptive push buttons.

The fastening tabs are joined with the side portions of the absorbent article in connection areas, preferably being placed in the parts of the side portions situated at the side edge extending along the longitudinal direction of the side portions. The connection areas are the part of the fastening tab and the part of the side portion being joined together. The joint can be achieved by for instance adhesive bonding, tape, thermal sealing or welding in the connection area, where the joint itself can be designed be bonding in discrete points, lines or continuous surfaces. Preferably, the edge of the connection area being closest to the side edge extending along the longitudinal direction is parallel or essentially parallel to the side edge of the side portion. It is also possible to let the edge of the connection area and the side edge of the side portion coincide. Naturally, the edge of the connection area can be designed so that it is not being parallel to the side edge of the side portion in that the edge exhibit a beam shaped or wave shaped line.

The fastening tabs can be attached on the back sheet, top sheet, between the back sheet and the top sheet of the absorbent article or be designed that the tabs at the connection area is attached so as the part of the tab being on the outside of the back sheet and another part of the tab on the inside of the article, i.e., on the side of the top sheet being faced against the wearer. Another possibility is to provide the fastening tab with a tape layer at the part of the tab, which at application of the tab on the article comprise the connection area.

The width of the fastening tab in the transversal direction, extending from the edge of the connection area being closest to the fastening means of the fastening tab, to the part of the fastening means, being closest to the attachment of the fastening tab is defined as the projecting part U of the fastening tab. The projecting part U together with the height H of the fastening tab along the projecting part is significant to estimate the stability of the fastening of the absorbent article about the user. The height of the fastening tab can be described as the elongation along the projecting part of the fastening tab in a direction being perpendicular or essentially perpendicular to the projecting part U and coincides or substantially coincides with the longitudinal direction of the article. The height can vary along the projecting part depending of which design of the fastening tab being desirable. Since the height of the fastening tab does not have to be constant along the width of the fastening tab, it is therefore the smallest height H of the fastening tab along the projecting part being of the greatest importance for the stability.

In order to provide enough stability of the absorbent article about the wearer, in that the article not has a tendency to twist or loosen around the waist, the fastening tab should be designed to achieve a good stability. To obtain a good stability of the fastening tab being of the type of soft and inelastic fastening tabs of the present invention, the relationship between the therefore the smallest height H of the fastening tab along the projecting part and the projecting part U to obtain enough stability of the tab, must at least be such that the therefore the smallest height H along the projecting part in relationship to the projecting part U is greater than a certain value. Tests have proven that enough stability is obtained when the relationship H/U has a value such that H/U > 3.

Upon application of the absorbent article on a user, the absorbent article is being placed between the legs of a user in the crotch area of the user. Thereafter, the article is closed about the user by stretching the rear side portions about the waist of the user as to apply the fastening means of the fastening tabs against reception portion of the front portion of the article to attach the article on the user.

### Brief description of the drawings

The invention will in the following be closely described with reference to the embodiments shown on the drawings.
- Figure 1: shows a planar view of a diaper according to the invention.
- Figure 2: shows a sectional view along the line II-II in figure 1.
- Figures 3a-3c: show sectional view along the line III-III in figure 1.
- Figure 4: shows a first embodiment of a fastening tab.
- Figure 5: shows a second embodiment of a fastening tab.
- Figure 6: shows a third embodiment of a fastening tab.

### Description of embodiments

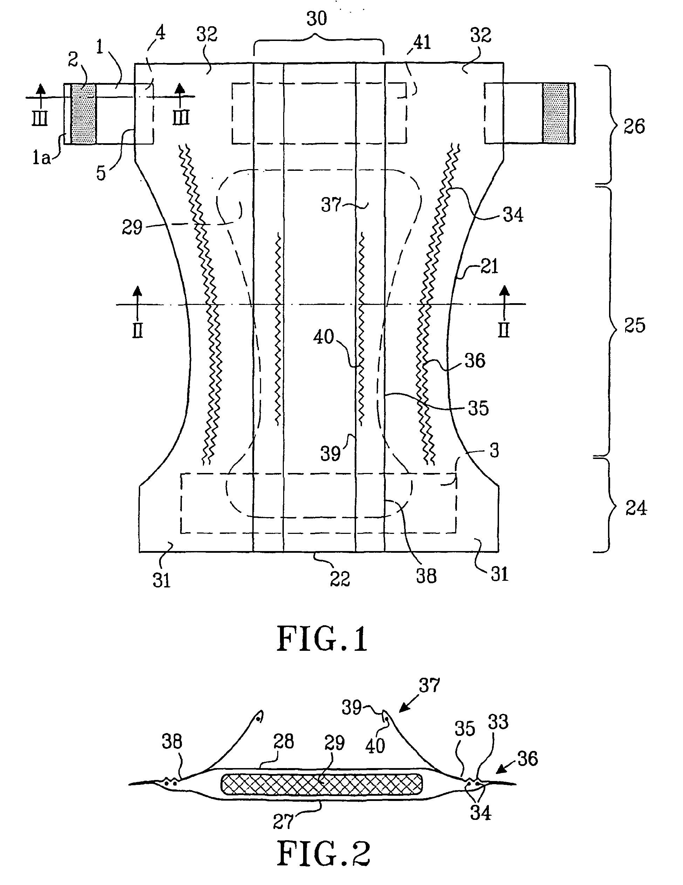

The diaper shown in figure 1 and in figure 2 has an essentially elongated shape having a longitudinal direction and a transversal direction and exhibits two side edges 21 extending along the longitudinal direction, one front end edge 22 extending in the transversal direction, one rear end edge 23 extending in the transversal direction, and a front portion 24, a rear portion 26 and a crotch portion 25 arranged therebetween. The diaper comprises a lower liquid impervious back sheet 27 faced from the wearer, an upper liquid permeable top sheet 28 faced against the wearer and an absorption core 29 arranged between said sheets. The absorption core is elongated in the longitudinal direction and constitute together with the directly adjacent portions of the back sheet and the top sheet one in the longitudinal direction extending central portion 30. On each sides of the central portion 30, the article exhibits front side portions 31 and rear side portions 32 at the front and rear portions, respectively. The absorbent core 29 has an extended shape in the longitudinal direction and is essentially hourglass shaped.

The back sheet 27 extends along total side of the diaper being faced away from the user during use. The top sheet 28 extends along the central portion 30 and essentially covers the absorbent core 29. On each side of the top sheet 28 in the transversal direction on the side of the diaper being faced against the user, side sheets 33 extend, which can be seen as an extension of the top sheet. The side sheets 33 is preferably essentially liquid impervious and in the area outside the absorbent core 29, the back sheet 27 and the liquid impervious side sheets 33 are connected to each other using for example adhesive or by thermal bonding. In the crotch portion 25 elastic means 34 are arranged between the back sheet 27 and the side sheets 33 on each sides of the absorbent core 29. The elastic means 34 functions as leg elastic and are arranged to prevent liquid and faeces from leaking out from side edges 21 extending in the longitudinal direction and in that way forms outer liquid barriers 36 together with the ambient sheets. The elastic means consist of one or many elastic threads, which in a stretched condition have been applied on the diaper. The side sheets 33 are connected to the top sheet 28 using for instance ultrasonic welding in the areas 35, extending along the diaper in the longitudinal direction.

In order to further prevent liquid or faeces from leaking ont, the diaper is also provided with inner liquid barriers 37 on the side facing the user, which are extensions of the side sheets 33 and extends beyond the connections 35 connecting the side sheets 33 with the top sheet 28. The inner liquid barriers 37 are folded down in the front and rear portions of the diaper and are attached against the top sheet 28. In the crotch portion 25, the inner liquid barriers 37 have a first part 38 being connected to the top sheet 28 by connections 35 between the top sheet and the side portions and a second free edge 39 intended to lye close to the user during usage. The other free edge 39 is provided with an elastic thread 40, which in a contracted condition contracts the free edge 39 creating a standing barrier in the crotch portion. The inner barrier 37 is formed of a part of the side sheets 33, wherein the free edge is folded to enclose the elastic thread 40, to prevent a direct contact of the elastic thread against the user. Instead of having separate side sheets, it is also possible to form extensions of the top sheet or the back sheet.

The rear and/or the front portions of the diaper can also be provided with a so-called waist elastic, consisting of elastic means arranged along the front end edges and/or rear end edges in order to provide a soft and flexible enclosure around the waist of the user. In this embodiment, only the rear portion 26 is provided waist elastic 41, being a thin ribbon of an elastic foam material being attached using an adhesive between the back sheet and the top sheet. The waist elastic 41 is applied in a prestretched condition between the layers to obtain a contracting force, extending around the waist of the user.

Two soft and inelastic fastening tabs 1 are arranged on the rear side portions 32 in order to attach the absorbent article around a wearer, having one tab on each side portion. The fastening tab joins the rear and front portions to each other, in that the fastening means 2 arranged at the fastening tabs can attach against a reception portion 3 for the fastening means 2 arranged at the front portion of the diaper. The fastening tabs 1 are made of a very soft and inelastic material such as a single nonwoven layer or a laminate.

Preferably, the fastening means 2 may consist of a hook portion of a hook and loop type fastener and is attached on the part of the fastening tab which is faced away from the side portions of the article and on the side of the fastening tab which is faced against the reception portion 3 during usage of the diaper. In order to obtain a good stability by the fastening of the diaper on the user, the fastening means 2 preferably has an extension in the longitudinal direction, which conforms to the height of the fastening tab in the longitudinal direction. If the height of the fastening tab varies, the extension of the fastening means 2 in the longitudinal direction should be equal to the smallest height of the fastening tab in said direction.

The reception portion 3 for the fastening tab 1 consist of a ribbon of a reception material suitable for the fastening means and which extends substantially parallel to the front end edge 22 on the side of the diaper being faced away from the wearer, that is, on the outside of the back sheet 27. The reception material in the present embodiment consist of a loop portion of a hook and loop type fastener and if preferably designed so that the extension in the longitudinal direction is in conformity with the fastening means.

Upon application of the diaper on a user, it is being placed between the legs of a user in the crotch area of the user. Thereafter, the article is closed about the user, in a way that the rear side portions 32 overlaps the front side portions 31 in that the front side portions are stretched around the waist of the user as to permit the application of the fastening means 2 of the fastening tabs 1 against the reception portion 3 to attach the article.

In order to be able to easily loosen the fastening tabs 1 from the reception portion 3, a part of the tabs 1 extend beyond the fastening means 2 and form gripping tabs 1a, which makes it easy to grab the tab 1. In the figures is gripping tabs shown having round edges but it is possible to vary the design of the gripping tabs la for them to be bevelled or another shape.

The fastening tabs 1 are connected to the rear side portion 32 in the connection areas 4 being placed in these areas of the side portions being at the side edge 21 extending in the longitudinal direction of the side portions 32. The connection areas consist of the part of the fastening tab 1 and the part of the side portion 32 being connected to each other. Preferably, the edges of the connection areas being closest to the side portions being at the side edge 21 extending in the longitudinal direction of the side portions 32 are parallel or essentially parallel to the side edge of the side portions. It is also possible to let the edge of the connection area and the edge of the side portion coincide.

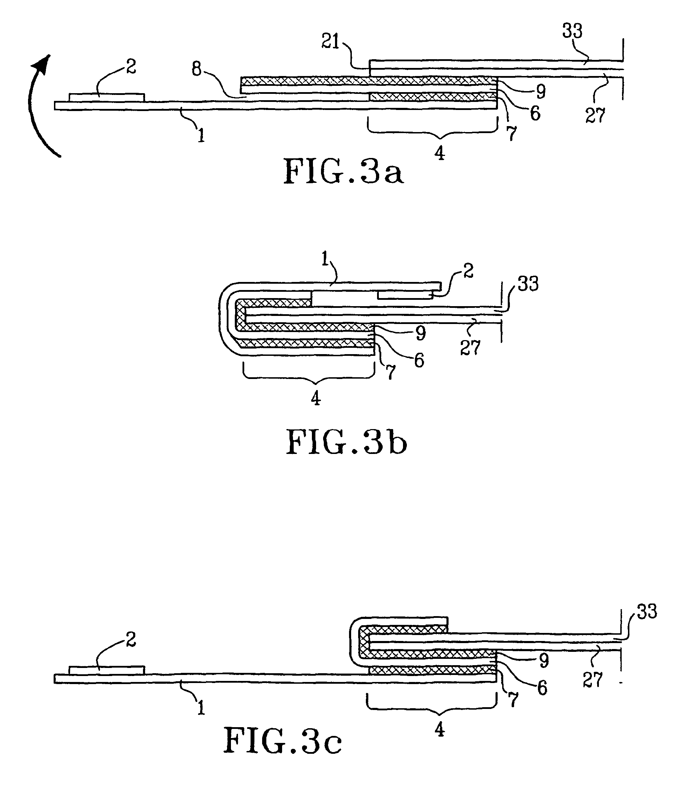

According to a preferred embodiment, the fastening tab 1 are provided with a tape layer 6 at the part of the tab comprising the connection area 4 upon application of the tabs on the article, see figure 3a. The surface of the tape layer 6 being faced against the fastening tabs 1 has a area being closest to the edges of the tabs against the side portion being provided with a first adhesive layer 7 for the attachment of the tape layer 6 on the tabs 1 and a surface lacking adhesive 8 at the part of the tape layer being faced against the fastening means of the tabs. The total surface of the tape layer 6 being faced away from the tabs and thus comprise the surface, which will attach the tabs on the diaper, is provided with a second adhesive layer 9. Upon application of the tab on the diaper, it is appropriate to begin from the tabs where the tape layer 6 already is attached on the tabs by the first adhesive layer 7 and then apply the tabs on the diaper as to attach the surface of the second adhesive layer 9 essentially corresponding to the surface of the first adhesive layer 7 on the back sheet 27 at the rear side portions 32. The side edges 21 extending in the longitodinal direction of the side portions 32 should conform or substantially conform with the line delimiting the first adhesive layer 7 of the tape layer 6 and a surface lacking adhesive 8 as to permit folding of the fastening tabs about the side edge of the rear side portions as to permit the attachment of the part of the second layer 9 which does not attach to the back sheet 27 against the side sheets 33, see figure 3b. In this way, a stable attachment of the fastening tab is obtained, at the same time being suitable from a manufacturing view. Since the tab is folded against the side sheet 33 of the article, it is also not in the way at the folding and packaging of the diaper. During usage of the article, the tab is folded out before the fastening means is connected to the reception portion, see figure 3c.

In order to obtain a good enough stability for the type of soft-inelastic fastening tabs of the present invention, the relationship between the smallest height H of the fastening tab along the projecting part and the projecting part U, shall at least be such that H/U >3, as previously described.

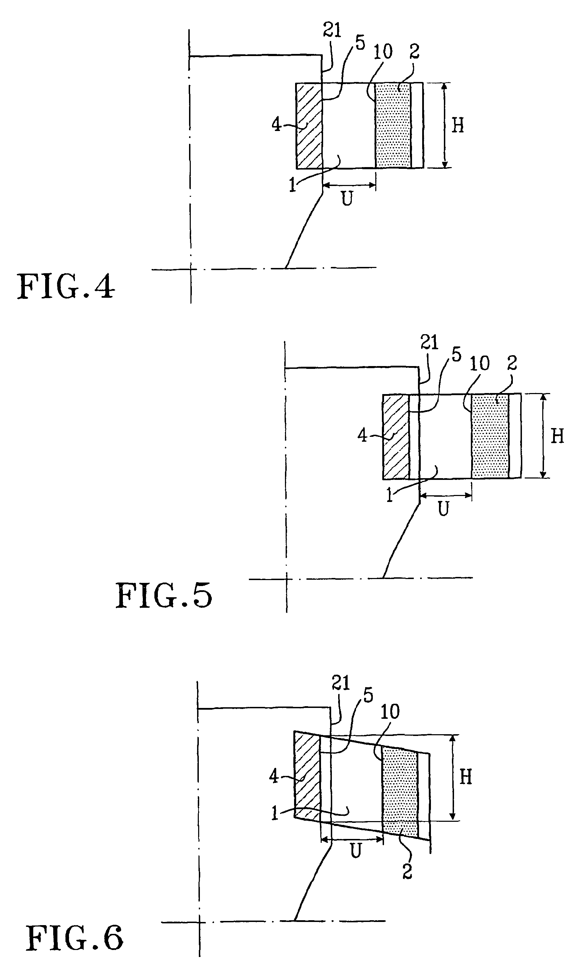

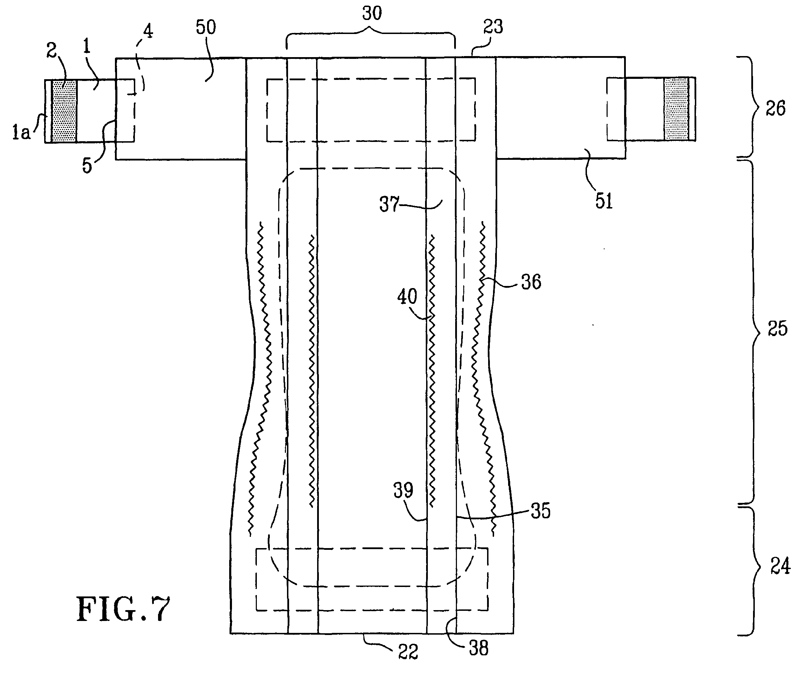

In the embodiments according to the figures 4 and 5, the fastening tabs 1 are provided with mutually straight or essentially straight edges in both the longitudinal direction as well as in the transversal direction and in figure 6 the fastening tabs 1 are diagonal but with mutually straight or essentially straight edges. The tabs are connected with the rear side portions 32 in the connection areas 4, each having an edge 5 being closest to the side edge 21 of the rear side portion in the longitudinal direction. In the embodiments according to figure 5 and figure 6, the edges 5 of the connection areas 4 are placed on a distance within the side edges 21 of the rear side portions and in the embodiment according to figure 4 placed so that the edges 5 coincide with the side edges 21 of the rear side portions32, in the same way as previously described in connection to the figures 3a-3c.

In the embodiments according to the figures 4-6, the projecting part U, which has been previously defined, the width of the fastening tab 1, extending from the edge 5 of the connection area closest to the side edge 21 extending in the longitudinal direction of the rear side portion 32 to the edge 10 of the fastening means 2, which is closest to the attachment of the fastening tab. The smallest height H is the smallest height of the fastening tab along the projecting part in a direction being substantially perpendicular to the projecting part U.

In the same way as previously, the projecting part U is determined as the width of the fastening tab in the transversal direction extending from the edge 5 of the connection area, which in the transversal direction is closest to fastening means 2 of the fastening tab 1 to the edge 10 of the fastening means which is closest to the attachment of the fastening tab. The smallest height H of the fastening tab, is in the same way as previously described the smallest height of the fastening tab along the projecting part in direction being perpendicular or substantially perpendicular to the projecting part U.

The design of the fastening tabs is not limited to the above-described embodiments but can be designed in a way, being suitable for the use of absorbent article. They may for instance have rounded edges or edges exhibiting an angle in different ways in relation to the article. It is further possible to have more than one fastening tab on each side of the article.

The invention shall not be regarded as being limited to the described embodiments since these are only intended to clarify the invention. Within the scope of the invention, it is possible to combine characteristics from the different embodiments with each other.

## Claims

1. Fastening means for the fastening of an absorbent article on a user, said article having a longitudinal and a transverse direction, said fastening means comprising at least one soft and inelastic fastening tab (1) and one fastening means (2) arranged on the fastening tab (1), where the fastening tab (1) at one of its ends is joined to any of the side portions (32) of the article, in connection areas (4) being the part of the fastening tab (1) and the part of the side portion (32) which are connected to each other, said connection areas (4) having an edge (5) which in the transversal direction is located closest to the fastening means (2), whereby the fastening tab (1) in the longitudinal direction of the article has a smallest height, H, and a projecting part, U, being the width of the fastening tab in the transversal direction of the article extending from the edge (5) of the connection area (4) to the portion (10) of the fastening means (2) being closest to the connection area (4), ***characterised in*, *that*** the relationship between smallest height H of the fastening tab along the projecting part of the fastening tab and the projecting part U of the fastening tab is such that H/U > 3 and that the fastening tab has a Gurley stiffness being lower than 100 mg.

2. Fastening means according to any of the preceding claims, **characterised in, that** the fastening tab has a Gurley stiffness being lower than 75 mg.

3. Fastening means according to any of the preceding claims, **characterised in, that** the fastening means (2) has an extension in the longitudinal direction which at least equals the smallest height H of the fastening tab (1).

4. Absorbent article comprising a liquid impervious back sheet (27), a liquid permeable top sheet (28) and an absorbent core (29) arranged between the back sheet and the top sheet, **characterised in, that** the absorbent article comprises a fastening means according to any of the claims 1-3.

5. Absorbent article according to claim 4, **characterised in, that** it is provided with two fastening tabs (1), which are joined to the absorbent article at its rear side portions (32).

## Revendications

1. Moyen de fixation destiné à la fixation d'un article absorbant sur un utilisateur, ledit article comportant une direction longitudinale et une direction transversale, ledit moyen de fixation comprenant au moins une patte de fixation (1) souple et non élastique et un moyen de fixation (2) agencé sur la patte de fixation (1), la patte de fixation (1) étant réunie à une de ses extrémités à l'une quelconque des parties latérales (32) de l'article dans des zones de connexion (4) qui sont la portion de la patte de fixation (1) et la portion de la partie latérale (32) qui sont connectées l'une à l'autre, lesdites parties de connexion (4) comportant un bord (5) qui, dans la direction transversale, est situé très près du moyen de fixation (2), grâce à quoi la patte de fixation (1) a une très faible hauteur (H) dans la direction longitudinale de l'article, et une partie en saillie (U) étant la largeur de la patte de fixation dans la direction de l'article, s'étendant depuis le bord (5) de la zone de connexion (4) jusqu'à la partie (10) du moyen de fixation (2) qui est très proche de la zone de connexion (4) ***caractérisé en ce que*** la relation entre la plus faible hauteur (H) de la patte de fixation le long de la partie en saillie de la patte de fixation et la partie en saillie (U) de la patte de fixation est telle que H/U > 3 et que la patte de fixation a une rigidité Gurley qui est inférieure à 100 mg.

2. Moyen de fixation selon l'une quelconque des revendications qui précèdent, **caractérisé en ce que** la patte de fixation a une rigidité Gurley inférieure à 75 mg.

3. Moyen de fixation selon l'une quelconque des revendications qui précèdent, **caractérisé en ce que** le moyen de fixation (2) a une extension dans la direction longitudinale qui est au moins égale à la plus faible hauteur (H) de la patte de fixation (1).

4. Article absorbant comprenant une feuille arrière (27) imperméable aux liquides, une feuille supérieure (28) perméable aux liquides et un noyau absorbant (29) agencé entre la feuille arrière et la feuille supérieure, **caractérisé en ce que** l'article

5. Article absorbant selon la revendication 4, **caractérisé en ce qu'**il est muni de deux pattes de fixation (1) qui sont réunies à l'article absorbant dans les parties latérales arrière (32) de celui-ci.

## Patentansprüche

1. Befestigungseinrichtung zum Befestigen eines absorbierenden Gegenstands an einem Benutzer, mit einer Längs- und einer Querrichtung, wobei die Befestigungseinrichtung umfasst: wenigstens einen weichen und unelastischen Befestigungsstreifen (1) und eine Befestigungseinrichtung (2), die auf dem Befestigungsstreifen (1) angeordnet ist, wobei der Befestigungsstreifen (1) an einem seiner Enden mit einem beliebigen der Seitenabschnitte (32) des Gegenstands in Verbindungsbereichen (4) verbunden ist, die Teil des Befestigungsstreifens (1) und Teil des Seitenabschnitts (32) sind, die miteinander verbunden werden, wobei die Verbindungsbereiche (4) eine Kante (5) aufweisen, die in Querrichtung am nächsten zu der Befestigungseinrichtung (2) angeordnet ist, wobei der Befestigungsstreifen (1) in der Längsrichtung des Gegenstands eine geringste Höhe H aufweist, und sich ein vorragender Teil U, der die Breite des Befestigungsstreifens in Querrichtung des Gegenstands ist, von der Kante (5) des Verbindungsbereichs (4) zum Abschnitt (10) der Befestigungseinrichtung (2) am nächsten zum Verbindungsbereich (4) erstreckt, **dadurch gekennzeichnet, dass** die Beziehung zwischen der geringsten Höhe H des Befestigungsstreifens entlang des vorragenden Teils des Befestigungsstreifens und dem vorragenden Teil U des Befestigungsstreifens derart ist, dass H/U>3 und, **dadurch**, dass der Befestigungsstreifen eine Gurley-Steifigkeit von weniger als 100 mg aufweist.

2. Befestigungseinrichtung nach einem der vorstehenden Ansprüche, **dadurch gekennzeichnet, dass** der Befestigungsstreifen eine Gurley-Steifigkeit von weniger als 75 mg aufweist.

3. Befestigungseinrichtung nach einem der vorstehenden Ansprüche, **dadurch gekennzeichnet, dass** die Befestigungseinrichtung (2) eine Erstreckung in Längsrichtung aufweist, die wenigstens gleich der geringsten Höhe H des Befestigungsstreifens (1) ist.

4. Absorbierender Gegenstand, umfassend eine flüssigkeitsundurchlässige Decklage (27), eine flüssigkeitsdurchlässige Oberlage (28) und einen zwischen der Decklage und der Oberlage angeordneten Absorptionskern (29), **dadurch gekennzeichnet, dass** der absorbierende Gegenstand eine Befestigungseinrichtung gemäß einem der Ansprüche 1 bis 3 umfasst.

5. Absorbierender Gegenstand nach Anspruch 4, **dadurch gekennzeichnet, dass** er mit zwei Befestigungsstreifen (1) versehen ist, die mit dem absorbierenden Gegenstand an seinen hinteren Seitenabschnitten (32) verbunden sind.
